# EUROPÄISCHE PATENTSCHRIFT

(11) **EP 0 508 217 B1**
(45) Veröffentlichungstag und Bekanntmachung des Hinweises auf die Patenterteilung: **01.05.1996**
(21) Anmeldenummer: 92105103.3
(22) Anmeldetag: 25.03.1992
(51) Int. Cl.: C07D 213/61

(54) **Verfahren zur Herstellung von 2-Chlorpyridinen**
Process for the preparation of 2-chloropyridines
Procédé pour la préparation de chloro-2 pyridines

(30) Priorität: 06.04.1991 DE 4111214
(43) Veröffentlichungstag der Anmeldung: 14.10.1992
(73) Patentinhaber: BAYER AG, D-51368 Leverkusen (DE)
(72) Erfinder: Cramm, Günther, Dr., W-5090 Leverkusen 3 (DE); Lindel, Hans, Dr., W-5090 Leverkusen (DE)

(56) Entgegenhaltungen:
- EP-A- 0 004 414
- DE-A- 4 005 115
- DE-B- 1 178 052
- DE-B- 1 695 659
- SYNTHESIS. 1984, STUTTGART DE Seiten 743-746; T.R. KASTURI ET AL.: 'A Novel Transformation of 3-Alkoxyisoquinolines to 3-Chloroisoquinolines and an Unusual Decyanation of 1,3-Dialkoxy-4-cya no-5,6,7,8-tetrahydroisoquinolines Under Vilsmeier-Haack Conditions.'

## Beschreibung

Die vorliegende Anmeldung betrifft ein neues Verfahren zur Herstellung von 2-Chlorpyridinen.

Es ist bekannt, daß sich 2-Aminopyridine mit Hilfe der Sandmeyer-Reaktion oder ähnlich gelagerter Umsetzungen in die entsprechenden 2-Chlorpyridine überführen lassen. Gemäß DE-A 1 695 659 werden die besten Ausbeuten bei der Umsetzung einer gesättigten salzsauren methanolischen Lösung mit Alkylnitriten mit vorzugsweise 3 bis 5 Kohlenstoffatomen erhalten. Voraussetzung ist hier ein Molverhältnis 2-Aminopyridin zu Methanol von 1:8 bis 1:12.

Ein Nachteil des Verfahrens nach DE-A 1 695 659 ist die Tatsache, daß die bei der Umsetzung aus Alkylnitriten wie Butyl-, Amyl- oder Propylnitrit entstehenden Alkohole nur sehr aufwendig vom Methanol als Lösungsmittel zu trennen sind.

Es wurde nun gefunden, daß man 2-Chlorpyridin-Derivate der Formel (I) 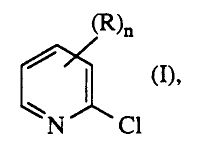
in welcher
- R: für Wasserstoff, C₁-C₄-Alkyl oder Halogen steht und
- n: für 1 bis 4 steht,
in guten Ausbeuten und hoher Reinheit erhält, wenn man
ein 2-Aminopyridin-Derivat der Formel (II) 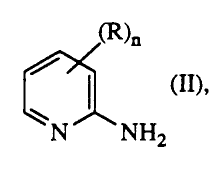
in welcher
R und n die oben angegebenen Bedeutungen haben,
in mit Chlorwasserstoff gesättigter Lösung bei Temperaturen zwischen -10°C und +50°C in einem Lösungsmittel der Reihe Methanol, Wasser oder einem Gemisch aus Methanol und Wasser mit Methylnitrit unter gleichzeitigem Einleiten von Chlorwasserstoff umsetzt.

Es ist als ausgesprochen überraschend zu bezeichnen, daß die anmeldungsgemäße Umsetzung mit Methylnitrit als Diazotierungsmittel durchgeführt werden kann, wird doch gemaß DE-A 1 695 659 ein Vorurteil dahingehend geschaffen, daß nur höhere Alkylnitrite mit vorzugsweise 3 bis 5 Kohlenstoffatomen für die Umsetzung geeignet erscheinen und daß die Umsetzung nur im absolutem Methanol durchführbar ist. Dieses Vorurteil wird durch das erfindungsgemäße Verfahren überwunden.

Die 2-Chlorpyridin-Derivate der Formel (I) können nach dem erfindungsgemäßen Verfahren auf relativ einfache Weise in sehr guter Ausbeute und hoher Reinheit gewonnen werden.

Die erfindungsgemäße Umsetzung wird vorzugsweise zur Herstellung von Verbindungen der Formel (I) angewandt, in welcher R für Wasserstoff, Chlor oder C₁-C₂-Alkyl steht und n für 1 oder 2 steht.

Nach dem erfindungsgemäßen Verfahren wird insbesondere die Verbindung 2-Chlor-5-methylpyridin aus 2-Amino-5-methyl-pyridin hergestellt.

Verwendet man 2-Amino-5-methyl-pyridin, HCl und Methylnitrit als Reaktionskomponenten, so kann der Reaktionsablauf wie folgt wiedergegeben werden:

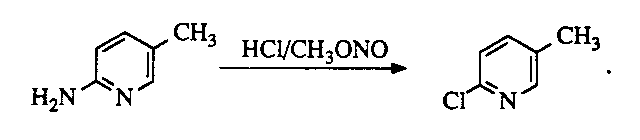

Die als Ausgangsverbindungen zu verwendenden 2-Aminopyridin-Derivate der Formel (II) sind bekannt.

Das zu verwendende Methylnitrit ist ebenfalls bekannt.

Als beim erfindungsgemäßen Verfahren einsetzbare 2-Aminopyridine der Formel (II) seien beispielhaft genannt:
2-Amino-5-methyl-pyridin, 2-Amino-3-methyl-pyridin, 2-Amino-4-methyl-pyridin, 2-Amino-6-methyl-pyridin, 2-Amino-pyridin, 2-Amino-4-chlor-pyridin, 2-Amino-5-ethyl-pyridin, 2-Amino-3,5-dimethyl-pyridin. Besonders bevorzugt wird 2-Amino-5-methyl-pyridin eingesetzt.

Das erfindungsgemäße Verfahren wird durchgeführt, indem man das 2-Aminopyridin-Derivat der Formel (II) in mit Chlorwasserstoff gesättigter methanolischer, wäßriger oder wäßrig-methanolischer lösung mit Methylnitrit begast. Hierbei wird gleichzeitig Chlorwasserstoffgas eingeleitet, so daß die Reaktionsmischung immer mit Chlorwasserstoff gesättigt ist.

Es werden bevorzugt 1 bis 5 Moläquivalente, insbesondere 1,1 bis 3 Moläquivalente Methylnitrit pro Mol Aminopyridin-Ausgangsverbindung (II), eingesetzt.

Das Verfahren wird im allgemeinen bei Temperaturen zwischen -10°C und +50°C, vorzugsweise zwischen 0°C und +20°C, durchgeführt.

Das Verfahren kann im absolutem Methanol, in reinem Wasser sowie in wäßrigen Methanol durchgeführt werden. Besonders bevorzugt wird als Reaktionsmedium wäßriges Methanol eingesetzt, wobei das Gewichts-Verhältnis Methanol:Wasser, vorzugsweise im Bereich von 8:1 bis 1:3 und insbesondere im Bereich von 3:1 bis 1:1 liegt.

Es wird im allgemeinen unter Normaldruck gearbeitet.

Nach beendeter Reaktion wird das Reaktionsgemisch durch Abdestillieren des eingesetzten Lösungsmittels bzw. Lösungsmittelgemisches eingeengt, anschließend wird durch den Zusatz wäßriger Basen (insbesondere Alkalihydroxide) alkalisch gestellt und die organische Phase abgetrennt. Aus der organischen Phase wird das 2-Chlorpyridin (I) vorzugsweise durch Destillation in reiner Form erhalten. Alternativ hierzu kann nach dem Alkalisch-Stellen das Reaktionsprodukt der Formel (I) auch durch Azeotrop-Destillation mit Wasser isoliert werden. Das dabei erhaltene Azeotrop kann vom Wasser getrennt und zusammen mit der obengenannten organischen Phase einer Feindestillation unterworfen werden.

Die Ausbeuten am Reaktionsprodukt 2-Chlor-pyridin-Derivat der Formel (I) betragen ohne Aufarbeiten der Nebenprodukte 80 bis 90 % der Theorie.

Bei der erfindungsgemäßen Umsetzung entstehen in geringen Mengen Nebenprodukte, so z.B. ein Pyridon-2-Derivat. Dieses befindet sich beim Aufarbeiten nach dem Abtrennen der organischen Phase in der Mutterlauge, wobei beim weiteren Einengen (Abdestillieren von Wasser) das Pyridon-2-Derivat auskristallisiert. Nach dem Abtrennen des Pyridon-2-Derivats kann dieses in an sich bekannter Weise, z.B. nach dem Verfahren der Deutschen Auslegeschrift Nr. 1 178 052 durch Umsetzung mit Thionylchlorid in Gegenwart eines N,N-dialkyl-substituierten Formamids in das entsprechende 2-Chlorpyridin-Derivat übergeführt werden. Dadurch läßt sich die Gesamtausbeute an 2-Chlorpyridin der Formel (I) nochmals steigern.

Diese Aufarbeitung der Pyridon-2-haltigen Mutterlauge kann als zusätzliche Verfahrensmaßnahme durchgeführt werden.

Bei der Umsetzung in methanolischer und methanolisch-wäßriger Lösung kann bei der Destillation der organischen Phase gegebenenfalls als Nebenprodukt der Umsetzung eine geringe Menge eines 2-Methoxy-pyridin-Derivats im Vorlauf der Destillation isoliert werden.

Aus diesem 2-Methoxy-pyridin kann nach an sich bekannter Methode (vgl. z.B. Synthesis 1984, 743) ebenfalls 2-Chlor-5-methyl-pyridin gewonnen werden.

Diese Aufarbeitung der 1. Fraktion der Destillation der organischen Phase kann ebenfalls als zusätzliche Verfahrensmaßnahme durchgeführt werden.

Das nach dem erfindungsgemäßen Verfahren herstellbare 2-Chlor-5-methyl-pyridin ist als Zwischenprodukt für Pharmazeutika bekannt (vgl. DE-A 2 812 585).

Weiterhin kann 2-Chlor-5-methyl-pyridin als Zwischenprodukt für die Herstellung von insektiziden Nitromethylen-Derivaten eingesetzt werden (vgl. EP-A 163 855).

Ferner können die erfindungsgemäß herstellbaren Verbindungen als Zwischenprodukte zur Herstellung von Diazotypiefarbstoffen (vgl. GB-A 870 027) und Haarfarbstoffen (vgl. DE-A 1 142 045) eingesetzt werden.

Schließlich sind die erfindungsgemäß herstellbaren 2-Chlorpyridine wertvolle Zwischenprodukte für die Herstellung von herbizid wirksamen α-[4-(Pyrid-2-yl-oxy)-phenoxy]-alkancarbonsäuren und deren Derivaten (siehe dazu CH-Patent Nr. 622 170).

### Herstellungsbeispiel 1

600 g 2-Amino-5-methylpyridin werden in 800 g Methanol gelöst und diese Lösung bei 10 bis 15°C mit Chlorwasserstoffgas gesättigt. Danach werden gleichzeitig 400 g Chlorwasserstoff und 360 g Methylnitrit bei maximal 15°C innerhalb von sechs Stunden gasförmig dem Reaktionsansatz zugeführt.

Nach einer Nachreaktionszeit von einer Stunde wird unter vermindertem Druck bei maximal 35°C das Lösungsmittel Methanol gemeinsam mit dem Chlorwasserstoff abdestilliert. Anschließend wird der Destillationsrückstand mit 500 ml Wasser verdünnt und bei maximal 80°C mit wäßriger Natronlauge auf pH 7 bis 8 gestellt. Die sich dabei als Oberphase abscheidende organische Phase, welche dem Hauptteil an 2-Chlor-5-methyl-pyridin enthält, wird abgetrennt.

Beim Andestillieren der wäßrigen Phase gehen geringe zusätzliche Mengen an 2-Chlor-5-methylpypridin als wäßriges Azeotrop über. Sie werden nach dem Abtrennen des Wassers mit der organischen Phase vereinigt und gemeinsam unter vermindertem Druck destilliert. Dabei werden 610 g (86,1 % der Theorie) 2-Chlor-5-methylpyridin (Kp₂₀ = 86°C) erhalten. In der ersten Fraktion der Destillation erhält man 62 g 2-Methoxy-5-methylpypridin (9,3 % der Theorie mit Kp₂₀ = 65°C). In der wäßrigen Mutterlauge verbleiben 25 g 5-Methyl-pyridon-2-.

Das Nebenprodukt 5-Methyl-pyridon-2 läßt sich durch fraktionierte Kristallisation abtrennen und nach bekanntem Verfahren (vgl. Deutsche Auslegeschrift 1 178 052) ebenfalls in 2-Chlor-5-methyl-pyridin überführen.

Die oben beschriebene, das 2-Methoxy-5-methyl-pyridin enthaltende Fraktion des Destillats der organischen Phase läßt sich ebenfalls nach bekannten Verfahren in 2-Chlor-5-methyl-pyridin überführen (vgl. Synthesis 1984, 743).

Die Gesamtausbeute an 2-Chlor-5-methyl-pyridin nach Aufarbeitung beider obengenannten Nebenprodukte beträgt 95 % der Theorie.

### Herstellungsbeispiel 2

Die Lösung von 540 g 2-Amino-5-methyl-pyridin in 500 g Wasser bzw. dem salzsauren wäßrigen Destillat eines vorausgegangenen Ansatzes (siehe unten) wird bei 0 bis 10°C mit Chlorwasserstoffgas gesättigt. Innerhalb von 6 Stunden wird die Lösung bei 0 bis 10°C simultan mit 335 g Methylnitrit und 450 g Chlorwasserstoff begast. Nach einer Nachreaktionszeit von 3 Stunden wird aus der Reaktionslösung zunächst unter vermindertem Druck bei 30°C das aus dem Methylnitrit entstandene Methanol gemeinsam mit Chlorwasserstoff abdestilliert. (Dieses Destillat wird beim nächsten Ansatz für die Synthese von Methylnitrit aus wäßriger NaNO₂-Lösung eingesetzt).

Anschließend werden ungefähr 500 g Wasser mit der Hauptmenge des freien Chlorwasserstoffs abdestilliert und beim folgenden Ansatz wieder als Reaktionsmedium verwendet.

Der Rückstand wird mit 500 g Wasser bzw. mit beim Einengen (siehe unten) erhaltenem Destillat verdünnt und mit 7 Mol Natronlauge bei 70 bis 80°C auf pH 8-9 gestellt. Die sich dabei abscheidende organische Phase wird abgetrennt und die NaCl-gesättigte, wäßrige Phase anschließend auf ein Viertel des Ausgangsvolumen eingeengt.

Dabei geht zunächst noch eine geringe Menge 2-Chlormethylpyridin als Azeotrop über. Es wird vom Wasser getrennt und zusammen mit der organischen Phase über eine Kolonne destilliert. Die Ausbeute an 2-Chlor-5-methylpyridin beträgt 520 g bzw. 82 % der Theorie.

Die beim Einengen der wäßrigen Phase erhaltenen ca. 500 g Wasser werden zum Verdünnen des folgenden Ansatzes vor der Neutralisation verwendet. Das nach Abdestillieren von weiteren ca. 250 g Wasser ausgefallene Kochsalz wird heiß abgesaugt. Das beim Abkühlen der Mutterlauge auskristallisierende 5-Methylpyridon-2 wird abgetrennt, die verbleibende Mutterlauge wird dem folgenden Ansatz nach der Phasentrennung zugesetzt und das restliche Pyridon durch Einengen der Mutterlauge wie zuvor beschrieben mitisoliert. Aus dem Nebenprodukt 5-Methyl-pyridon-2 läßt sich in an sich bekannter Weise (DE-AS 1 178 052) ebenfalls 2-Chlor-5-methylpyridin gewinnen.

### Herstellungsbeispiel 3

Die Lösung von 600 g 2-Amino-5-methylpyridin in 270 g Wasser und 480 g Methanol werden bei ca. 10°C mit Chlorwasserstoffgas gesättigt.

Innerhalb von 6 Stunden wird die Lösung bei ca. 10°C mit 380 g Methylnitrit und 500 g Chlorwasserstoff begast. Nach weiteren zwei Stunden wird unter vermindertem Druck zunächst bei maximal 35°C das Methanol gemeinsam mit Chlorwasserstoff und anschließend bei max. 50°C noch ca. 100 g wäßriges, salzsaures Destillat abdestilliert.

Anschließend wird der Destillationsrückstand mit ca. 500 ml Wasser verdünnt, mit Natronlauge auf pH 8-9 gestellt und die organische Phase abgetrennt.

Aus der wäßrigen Phase wird das restliche 2-Chlor-5-methylpyridin durch Wasserdampfdestillation abgetrennt.

Nach destillativer Aufarbeitung werden insgesamt 602 g 2-Chlor-5-methylpyridin (84,9 % der Theorie) und 59 g 2-Methoxy-5-methylpyridin (8,6 % der Theorie) erhalten. 31 g 5-Methylpyridon-2 (5 % der Theorie) lassen sich aus der Mutterlauge durch fraktionierte Kristallisation isolieren. Aus den Nebenprodukten 2-Methoxy-5-methylpyridin und 5-Methylpyridon-2 läßt sich in an sich bekannter Weise ebenfalls 2-Chlor-5-methylpyridin herstellen (siehe dazu Hinweise in Herstellungsbeispiel 1).

## Patentansprüche

1. Verfahren zur Herstellung von 2-Chlorpyridin-Derivaten der Formel (I) 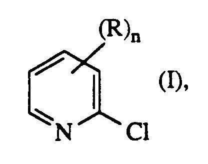 in welcher
R für Wasserstoff, C₁-C₄-Alkyl oder Halogen steht und
n für 1 bis 4 steht,
dadurch gekennzeichnet, daß man 2-Aminopyridin-Derivate der Formel (II) 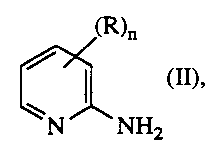 in welcher
R und n die oben angegebenen Bedeutungen haben,
in mit Chlorwasserstoff gesättigter Lösung bei Temperaturen zwischen -10°C und +50°C in einem Lösungsmittel der Reihe Methanol, Wasser oder einem Gemisch aus Methanol und Wasser mit Methylnitrit unter gleichzeitigem Einleiten von Chlorwasserstoff umsetzt.

2. Verfahren nach Anspruch 1, dadurch gekennzeichnet, daß man die Umsetzung bei 0°C bis 20°C durchführt.

3. Verfahren nach Anspruch 1 bis 2, dadurch gekennzeichnet, daß man 1 bis 5 Moläquivalente Methylnitrit pro Mol 2-Aminopyridin-Verbindung der Formel (II) einsetzt.

4. Verfahren nach Anspruch 1 bis 3, dadurch gekennzeichnet, daß man die Umsetzung unter Normaldruck durchführt.

5. Verfahren nach Anspruch 1 bis 4, dadurch gekennzeichnet, daß man nach beendeter Reaktion das Reaktionsgemisch durch Abdestillieren des Lösungsmittels einengt, anschließend durch Zusatz wäßriger Basen alkalisch stellt, die sich bildende organische Phase abtrennt und aus der organischen Phase das 2-Chlorpyridin (I) durch Destillation in reiner Form gewinnt.

6. Verfahren gemäß Anspruch 1 bis 5, dadurch gekennzeichnet, daß man nach beendeter Reaktion das Reaktionsgemisch durch Abdestillieren des Lösungsmittels einengt, anschließend durch Zusatz wäßriger Basen alkalisch stellt und das Reaktionsprodukt 2-Chlorpyridin-Derivat der Formel (I) durch Azeotropdestillation isoliert.

7. Verfahren gemäß Anspruch 1 bis 6, dadurch gekennzeichnet, daß man Verbindungen der Formel (I) herstellt, in welcher R für Wasserstoff, Chlor oder C₁-C₂-Alkyl und n für 1 oder 2 steht.

8. Verfahren gemäß Anspruch 1 bis 7, dadurch gekennzeichnet, daß man die Verbindung 2-Chlor-5-methyl-pyridin aus 2-Amino-5-methyl-pyridin herstellt.

9. Verfahren nach Anspruch 1 bis 8, dadurch gekennzeichnet, daß man das bei der Reaktion als Nebenprodukt entstehende Pyridon-2 nach an sich bekannten Methoden zum 2-Chlor-pyridin-Derivat der Formel (I) aufarbeitet.

10. Verfahren nach Anspruch 1 bis 9, dadurch gekennzeichnet, daß man die bei der Reaktion in Methanol oder Methanol/Wasser-Gemisch als Nebenprodukt entstehende 2-Methoxy-pyridin-Verbindung destillativ abtrennt und daraus nach an sich bekannten Methoden das 2-Chlor-pyridin-Derivat der Formel (I) herstellt.

## Claims

1. Process for the preparation of 2-chloropyridine derivatives of the formula (I) 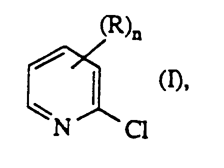 in which
R represents hydrogen, C₁-C₄-alkyl or halogen and
n represents 1 to 4,
characterised in that 2-aminopyridine derivatives of the formula (II) 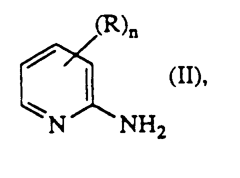 in which
R and n have the above definitions,
are reacted with methyl nitrite in hydrogen chloride-saturated solution at temperatures between -10°C and +50°C, in a solvent from the series comprising methanol, water or a mixture of methanol and water, with simultaneous introduction of hydrogen chloride.

2. Process according to Claim 1, characterised in that the reaction is carried out at 0°C to 20°C.

3. Process according to Claim 1 to 2, characterised in that 1 to 5 molar equivalents of methyl nitrite are used per mole of 2-aminopyridine compound of the formula (II).

4. Process according to Claim 1 to 3, characterised in that the reaction is carried out under atmospheric pressure.

5. Process according to Claim 1 to 4, characterised in that, when the reaction is complete, the reaction mixture is concentrated by distilling off the solvent, and then rendered alkaline by addition of aqueous bases, the organic phase that forms is removed, and the 2-chloropyridine (I) is obtained in pure form from the organic phase by distillation.

6. Process according to Claim 1 to 5, characterised in that, when the reaction is complete, the reaction mixture is concentrated by distilling off the solvent, and then rendered alkaline by addition of aqueous bases, and the reaction product 2-chloro-pyridine derivative of the formula (I) is isolated by azeotropic distillation.

7. Process according to Claim 1 to 6, characterised in that compounds of the formula (I) are prepared, in which R represents hydrogen, chlorine or C₁-C₂-alkyl and n represents 1 or 2.

8. Process according to Claim 1 to 7, characterised in that the compound 2-chloro-5-methyl-pyridine is prepared from 2-amino-5-methyl-pyridine.

9. Process according to Claim 1 to 8, characterised in that the 2-pyridone resulting as a by-product in the reaction is worked up to give the 2-chloro-pyridine derivative of the formula (I) by methods known per se.

10. Process according to Claim 1 to 9, characterised in that the 2-methoxy-pyridine compound resulting as a by-product in the case of the reaction in methanol or methanol/water mixture is removed by distillation, and the 2-chloro-pyridine derivative of the formula (I) is prepared therefrom by methods known per se.

## Revendications

1. Procédé pour la préparation de dérivés de la 2-chloropyridine de formule (I) 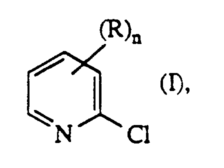 dans laquelle
R représente un atome d'hydrogène, un groupe alkyle en C₁-C₄ ou un atome d'halogène, et
n représente 1 à 4,
caractérisé en ce qu'on fait réagir des dérivés de 2-aminopyridine de formule (II) 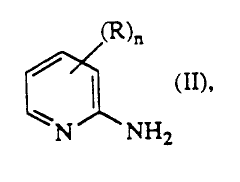 dans laquelle
R et n ont les significations indiquées ci-dessus,
dans une solution saturée avec de l'acide chlorhydrique, à des températures entre -10°C et +50°C, dans un solvant de la série du méthanol, de l'eau ou d'un mélange de méthanol et d'eau, avec du nitrite de méthyle, tout en introduisant simultanément de l'acide chlorhydrique.

2. Procédé selon la revendication 1, caractérisé en ce qu'on effectue la mise en réaction à une température de 0°C à 20°C.

3. Procédé selon les revendications 1 et 2, caractérisé en ce qu'on met en oeuvre de 1 à 5 équivalents molaires de nitrite de méthyle par mole du composé de 2-aminopyridine de formule (II).

4. Procédé selon les revendications 1 à 3, caractérisé en ce qu'on effectue la mise en réaction sous pression normale.

5. Procédé selon les revendications 1 à 4, caractérisé en ce que, au terme de la réaction, on concentre le mélange réactionnel par séparation du solvant par distillation, on règle ensuite le mélange pour le rendre alcalin par addition de bases aqueuses, on sépare la phase organique qui se forme et on obtient par distillation à partir de la phase organique la 2-chloropyridine (I) sous forme pure.

6. Procédé selon les revendications 1 à 5, caractérisé en ce qu'au terme de la réaction, on concentre le mélange réactionnel par séparation du solvant par distillation, ensuite on règle le mélange alcalin par addition de bases aqueuses et on isole le produit réactionnel, le dérivé de 2-chloropyridine de formule (I) par distillation azéotrope.

7. Procédé selon les revendications 1 à 6, caractérisé en ce qu'on prépare des composés de formule (I) dans laquelle R représente un atome d'hydrogène, un atome de chlore ou un groupe alkyle en C₁-C₂ et n représente 1 ou 2.

8. Procédé selon les revendications 1 à 7, caractérisé en ce qu'on prépare le composé de 2-chloro-5-éthyl-pyridine à partir de la 2-amino-5-méthyl-pyridine.

9. Procédé selon les revendications 1 à 8, caractérisé en ce qu'on traite la pyridone-2, que l'on obtient comme sous-produit lors de la réaction, conformément à des procédés connus en soi pour obtenir le dérivé de 2-chloropyridine de formule (I).

10. Procédé selon les revendications 1 à 9, caractérisé en ce qu'on sépare par distillation le produit de 2-méthoxypyridine que l'on obtient sous forme de sous-produit lors de la réaction dans du méthanol ou dans un mélange de méthanol/eau, et on prépare, à partir de là, conformément à des procédés connus, le dérivé de 2-chloropyridine de formule (I).
